# EUROPÄISCHE PATENTSCHRIFT

(11) **EP 3 861 096 B1**
(45) Veröffentlichungstag und Bekanntmachung des Hinweises auf die Patenterteilung: **16.10.2024**
(21) Anmeldenummer: 20700553.9
(22) Anmeldetag: 07.01.2020
(51) Int. Cl.: B01D 37/02, C12M 1/00, C12M 1/12, C12M 1/26

(54) **BIOPROZESSTECHNISCHE ANLAGE**
BIOPROCESSING INSTALLATION
INSTALLATION TECHNIQUE DE BIOPROCESSUS

(30) Priorität: 08.01.2019 DE 102019100339
(43) Veröffentlichungstag der Anmeldung: 11.08.2021
(73) Patentinhaber: Sartorius Stedim Biotech GmbH, 37079 Göttingen (DE)
(72) Erfinder: DIEL, Bernhard, 37079 Göttingen (DE)
(74) Vertreter: Novagraaf International SA
(86) Internationale Anmeldenummer: PCT/EP2020/050212
(87) Internationale Veröffentlichungsnummer: WO 2020/144184

(56) Entgegenhaltungen:
- EP-A1- 0 189 378
- EP-A1- 3 118 145
- DE-A1- 102006 052 669
- DE-A1- 102011 119 816
- DE-A1- 102016 000 979
- DE-A1- 2 721 226
- US-B1- 6 325 572

## Beschreibung

Die Erfindung betrifft eine bioprozesstechnische Anlage gemäß dem Oberbegriff von Anspruch 1, ein Verfahren für den Betrieb einer solchen bioprozesstechnischen Anlage gemäß dem Oberbegriff von Anspruch 13 sowie die Verwendung eines steril verpackten Transfersatzes für den Aufbau einer solchen bioprozesstechnischen Anlage gemäß dem Oberbegriff von Anspruch 15.

Unter einer bioprozesstechnischen Anlage wird hier ganz allgemein eine Einrichtung verstanden, mit der biotechnologische Prozesse durchgeführt oder unterstützt werden können. Beispielsweise umfasst eine bioprozesstechnische Anlage einen Bioreaktor, dem ein sogenanntes Downstream-System für das Ableiten und gegebenenfalls Weiterbehandeln der in dem Bioreaktor erzeugten Fermentationsbrühe nachgeschaltet ist.

Bei der bekannten bioprozesstechnischen Anlage (DE 10 2011 119 816 A1), von der die Erfindung ausgeht, ist zwischen dem Bioreaktor und einer Filteranordnung zur Filterung der Fermentationsbrühe eine Hauptleitung vorgesehen, die den Hauptstrom aus Fermentationsbrühe führt. Die Filteranordnung dient hier zur Zellabtrennung und arbeitet nach dem Prinzip der Anschwemmfiltration, für die ein Filterhilfsmittel in die zu filternde Fermentationsbrühe zu suspendieren ist, bevor die mit dem Filterhilfsmittel angereicherte Fermentationsbrühe als Suspension in die Filteranordnung einläuft.

Bei der bekannten bioprozesstechnischen Anlage ist für das Einbringen des Filterhilfsmittels ein Mischbeutel vorgesehen, der in eine Rezirkulationsleitung geschaltet ist. Die Verwendung eines solchen Mischbeutels ermöglicht eine staubfreie und weitgehend kontaminationsfreie Handhabung des Filterhilfsmittels. Der Mischbeutel und ein Großteil der übrigen Anlagenkomponenten lassen sich als Single-Use-Komponenten, also als Einwegkomponenten, ausbilden. Bei entsprechendem Austausch der Single-Use Komponenten reduziert ein solches Single-Use-Konzept grundsätzlich das Risiko einer Kreuzkontamination zwischen zwei aufeinanderfolgenden Batch-Prozessen.

Allerdings ergeben sich Herausforderungen im Hinblick auf die Prozessautomatisierung bei der Zugabe des Filterhilfsmittels, was bei der bekannten bioprozesstechnischen Anlage manuell vonstattengeht. Der hiermit verbundene manuelle, insbesondere körperliche, und zeitliche Aufwand ist vergleichsweise hoch, da das Filterhilfsmittel bei der bekannten bioprozesstechnischen Anlage in einer Vielzahl von Einzelchargen manuell gehandhabt werden muss.

Herausforderungen ergeben sich aber auch im Hinblick auf die Skalierbarkeit der Menge des zu transferierenden Filterhilfsmittels, die bei der bekannten bioprozesstechnischen Anlage aufgrund der begrenzten Anzahl von Nachfüllzyklen und aufgrund des begrenzten Aufnahmevolumens des Mischbeutels beschränkt ist. Herausforderungen ergeben sich schließlich im Hinblick auf die Regelbarkeit des Suspendierens des Filterhilfsmittels in der Fermentationsbrühe, da das Suspendieren im Mischbeutel bei der bekannten Anlage zu einer trägen Systemreaktion auf eine gewünschte Änderung der Konzentration des Filterhilfsmittels in der Fermentationsbrühe führt.

Zwar sind mehrere Pulvertransfersysteme bekannt, die grundsätzlich eine automatisierte Abgabe von pulverförmigen Medien erlauben. Allerdings handelt es sich dabei entweder um eine Abgabe aus einem Pulverbehälter in einen anderen Pulverbehälter (EP 3 118 145 A1) oder um die Abgabe aus einem Pulverbehälter in einen Flüssigkeitsbehälter (US 6,325,572 B1), sodass diese bekannten Pulvertransfervorrichtungen, soweit sie zur Anwendung auf die in Rede stehende, bioprozesstechnische Anlage überhaupt geeignet sind, den obigen Herausforderungen an die Prozessautomatisierung nicht gerecht werden. Ferner sind im Stand der Technik Transportsysteme für Pulver mit Kupplungseinrichtungen offenbart (DE 10 2016 000 979 A1).

Der Erfindung liegt das Problem zugrunde, die bekannte bioprozesstechnische Anlage derart auszugestalten und weiterzubilden, dass unter Beibehaltung des Single-Use-Konzepts die Prozessautomatisierung optimiert wird. Weiterhin ermöglicht die Erfindung eine, insbesondere staubfreie, Pulverzugabe, die nahezu ohne manuelle Arbeitsschritte erfolgt. Zudem kann die Prozesszeit und der Platzbedarf der bekannten bioprozesstechnischen Lösung verkürzt bzw. verringert werden.

Das obige Problem wird bei einer bioprozesstechnischen Anlage gemäß dem Oberbegriff von Anspruch 1 durch die Merkmale des kennzeichnenden Teils von Anspruch 1 gelöst.

Wesentlich ist die grundsätzliche Überlegung, dass die Hauptleitung, die an sich dem Leiten des Hauptstroms aus Fermentationsbrühe oder einem anderen flüssigen, insbesondere biologischen, Medium dient, genutzt werden kann, um das Filterhilfsmittel oder einen anderen Zusatz in dem flüssigen Medium bzw. der Fermentationsbrühe zu suspendieren oder zu lösen. Besonders vorteilhaft dabei ist die Tatsache, dass der ohnehin vorhandene Strömungszustand in der Hauptleitung genutzt werden kann, um das Vermischen zwischen dem Zusatz bzw. Filterhilfsmittel und dem flüssigen Medium bzw. der Fermentationsbrühe zu unterstützen.

In gleicher Weise könnte durch die Hauptleitung als flüssiges Medium auch eine beliebige andere Flüssigkeit wie z.B. Leitungswasser, steriles Wasser, eine Pufferlösung oder dergleichen geleitet werden und/oder als pulverförmiger Zusatz könnte ein zu lösender Stoff wie z.B. Puffersalz oder pulverförmiger Nährstoff vorgesehen sein.

Stromabwärts der Zulaufstelle für den Zusatz ist als ein mit dem flüssigen Medium beaufschlagbares Anlagenteil, das insbesondere von dem flüssigen Medium durchströmbar ist oder in das das flüssige Medium einströmen kann, vorzugsweise eine Filteranordnung vorgesehen. Auch könnte stromabwärts der Zulaufstelle und nach dem Lösen oder Suspendieren des Zusatzes, zusätzlich oder alternativ zu einer Filteranordnung, auch ein anderes mit dem flüssigen Medium beaufschlagbares Anlagenteil, insbesondere ein Aufnahmebehälter wie ein Vorlage- oder Mischbehälter zur Aufnahme des soeben gelösten oder suspendierten Gemisches angeordnet sein.

Im Einzelnen wird zunächst ganz allgemein vorgeschlagen, dass die bioprozesstechnische Anlage ein Pulvertransfersystem zur, insbesondere staubfreien, dosierten Abgabe eines Zusatzes und eine an das Pulvertransfersystem angeschlossene Zulaufleitung aufweist, die an einer Zulaufstelle in die Hauptleitung mündet, wobei die Zulaufleitung einen Rücklaufverhinderer zum Verhindern eines Rücklaufs von flüssigem Medium aufweist. Vorzugsweise ist vorgesehen, dass die bioprozesstechnische Anlage ein Pulvertransfersystem zur, insbesondere staubfreien, dosierten Abgabe eines Filterhilfsmittels und eine an das Pulvertransfersystem angeschlossene Zulaufleitung aufweist, die an einer Zulaufstelle in die Hauptleitung mündet, wobei die Zulaufleitung einen Rücklaufverhinderer zum Verhindern eines Rücklaufs von Fermentationsbrühe aufweist.

Dabei ist die Zulaufleitung eine Leitung im eigentlichen Sinne, die dafür ausgelegt ist, einen Fluidstrom zu leiten.

Wesentlich ist weiter, dass das Pulvertransfersystem den Zusatz in die Zulaufleitung in Pulverform einspeist und mittels eines druckbeaufschlagten Transportgases zu der Zulaufstelle treibt. Die Nutzung des druckbeaufschlagten Transportgases für den Transport des Zusatzes ermöglicht einerseits ein prozesssicheres Passieren des Rücklaufverhinderers durch den Zusatz und andererseits ein prozesssicheres Einspeisen des Zusatzes in die Hauptleitung, die den Hauptstrom aus flüssigem Medium führt. Vorzugsweise ist vorgesehen, dass das Pulvertransfersystem das Filterhilfsmittel in die Zulaufleitung in Pulverform einspeist und mittels eines druckbeaufschlagten Transportgases zu der Zulaufstelle treibt. Die Nutzung des druckbeaufschlagten Transportgases für den Transport des Filterhilfsmittels ermöglicht in diesem Fall einerseits ein prozesssicheres Passieren des Rücklaufverhinderers durch das Filterhilfsmittel und andererseits ein prozesssicheres Einspeisen des Filterhilfsmittels in die Hauptleitung, die den Hauptstrom aus Fermentationsbrühe führt.

Vorschlagsgemäß wird zumindest ein Teil des so eingespeisten Zusatzes innerhalb der Hauptleitung stromabwärts der Zulaufstelle in das flüssige Medium eingegeben, bevor das mit dem Zusatz angereicherte flüssige Medium das an die Hauptleitung angeschlossene Anlagenteil durchläuft oder erreicht. Vorzugsweise wird zumindest ein Teil des so eingespeisten Filterhilfsmittels innerhalb der Hauptleitung stromabwärts der Zulaufstelle in der Fermentationsbrühe suspendiert, bevor die mit dem Filterhilfsmittel angereicherte Fermentationsbrühe die Filteranordnung durchläuft.

Mit der vorschlagsgemäßen Lösung lässt sich dem flüssigen Medium ein pulverförmiger Zusatz staub- und kontaminationsfrei zugeben, da sich das Teilsystem zwischen dem Pulvertransfersystem und der Zulaufstelle auf einfache Weise geschlossen ausgestalten lässt. Vorzugsweise lässt sich der Fermentationsbrühe ein pulverförmiges Filterhilfsmittel staub- und kontaminationsfrei zugeben.

Ferner ist die Zugabe des Zusatzes nach der vorschlagsgemäßen Lösung ohne weiteres automatisierbar, da die dosierte Abgabe des Zusatzes allein auf eine entsprechende Ansteuerung einer dem Pulvertransfersystem zugeordneten Ventilanordnung zurückgeht. Vorzugsweise ist die Zugabe des Filterhilfsmittels nach der vorschlagsgemäßen Lösung ohne weiteres automatisierbar, da in diesem Fall die dosierte Abgabe des Filterhilfsmittels allein auf eine entsprechende Ansteuerung einer dem Pulvertransfersystem zugeordneten Ventilanordnung zur rückgeht.

Die vorschlagsgemäße Lösung ist im Hinblick auf die Menge des zuzugebenden Zusatzes frei skalierbar, da der Zusatz dem Pulvertransfersystem kontinuierlich zugeführt werden kann, ohne den Betrieb der bioprozesstechnischen Anlage unterbrechen zu müssen. Vorzugsweise ist die vorschlagsgemäße Lösung im Hinblick auf die Menge des zuzugebenden Filterhilfsmittels frei skalierbar, da in diesem Fall das Filterhilfsmittel dem Pulvertransfersystem kontinuierlich zugeführt werden kann, ohne den Betrieb der bioprozesstechnischen Anlage unterbrechen zu müssen.

Schließlich ist die Reaktionszeit der bioprozesstechnischen Anlage bei einer gewünschten Änderung der Konzentration des Zusatzes in dem flüssigen Medium außerordentlich gering, da der Zusatz vom Pulvertransfersystem direkt über die Zulaufleitung in die Hauptleitung, also ohne einen zwischengeschalteten Mischbehälter, eingespeist wird. Vorzugsweise ist die Reaktionszeit der bioprozesstechnischen Anlage bei einer gewünschten Änderung der Konzentration des Filterhilfsmittels in der Fermentationsbrühe außerordentlich gering, da in diesem Fall das Filterhilfsmittel vom Pulvertransfersystem direkt über die Zulaufleitung in die Hauptleitung, also ohne einen zwischengeschalteten Mischbehälter, eingespeist wird.

Aus alledem ergibt sich schließlich bei der vorschlagsgemäßen Lösung ein besonderer Vorteil im Hinblick auf das oben angesprochene Single-Use-Konzept.

Dadurch, dass mittels des Rücklaufverhinderers ein Rücklauf von flüssigem Medium bzw. Fermentationsbrühe in Richtung des Pulvertransfersystems ausgeschlossen ist, wird der Anschluss des Pulvertransfersystems an der Zulaufleitung stets frei von flüssigem Medium bzw. Fermentationsbrühe gehalten. Damit lässt sich im Sinne des Single-Use-Konzepts die Zulaufleitung zwischen zwei Batch-Prozessen austauschen, ohne dass das Pulvertransfersystem für den jeweils nächsten Batch-Prozess gereinigt werden muss. Die Zulaufleitung lässt sich also ohne weiteres als Single-Use-Komponente auslegen, während das Pulvertransfersystem ohne den üblicherweise erforderlichen Reinigungsaufwand mehrfach genutzt werden kann.

Gemäß Anspruch 2 ist vorzugsweise vorgesehen, dass das flüssige Medium Fermentationsbrühe ist, und/oder, dass das an die Hauptleitung angeschlossene Anlagenteil ein bioprozesstechnisches Funktionselement, vorzugsweise eine Filteranordnung zur Filterung der Fermentationsbrühe oder ein Aufnahmebehälter zur Aufnahme des flüssigen Mediums, ist, und/oder, dass der Zusatz ein Filterhilfsmittel und/oder Salz und/oder Nährstoff ist.

Anspruch 3 betrifft eine besonders bevorzugte Ausgestaltung, nach der das flüssige Medium Fermentationsbrühe ist, das an die Hauptleitung angeschlossene Anlagenteil eine Filteranordnung zur Filterung der Fermentationsbrühe und der Zusatz ein Filterhilfsmittel ist.

Bei der besonders bevorzugten Ausgestaltung gemäß Anspruch 4 weist die bioprozesstechnische Anlage einen Flüssigkeitsvorlagebehälter, insbesondere einen Bioreaktor, auf, wobei der Bioreaktor vorzugsweise, gemäß dem oben angesprochenen Single-Use-Konzept, einen zugeordneten Bioprozessbeutel aufweist. Grundsätzlich kann die bioprozesstechnische Anlage aber auch andere Komponenten wie beispielsweise Prozess-Mischsysteme, die zum Mischen von Pulvern, Suspensionen, Lösungen oder Emulsionen dienen können, aufweisen.

Gemäß Anspruch 5 kann das Single-Use-Konzept nicht nur die Zulaufleitung einschließlich des Rücklaufverhinderers, sondern auch die Hauptleitung einschließlich der Hauptstrompumpe umfassen. Dies betrifft grundsätzlich auch den Bioreaktor, sofern dieser einen zugeordneten Bioprozessbeutel aufweist.

Bei der weiter bevorzugten Ausgestaltung gemäß Anspruch 6 ist das Pulvertransfersystem mit einer Transportgasquelle für das druckbeaufschlagte Transportgas ausgestattet, bei der es sich um einen Druckbehälter für das Transportgas oder um eine Förderpumpe für das Transportgas handeln kann. Dabei kann die Einspeisung des Zusatzes bzw. Filterhilfsmittels in die Zulaufleitung diskontinuierlich oder kontinuierlich vorgesehen sein. Welche Betriebsweise hier gewählt wird, ist abhängig von der vorgegebenen Toleranz gegenüber Konzentrationsschwankungen des Zusatzes bzw. Filterhilfsmittels in dem flüssigen Medium bzw. der Fermentationsbrühe.

Der Rücklaufverhinderer der Zulaufleitung ist nach einer bevorzugten Variante von Anspruch 7 nach Art eines Rückschlagventils aufgebaut, bei dem ein sich verformender und/oder beweglicher Ventilkörper vorgesehen ist. Dies ermöglicht einen rein mechanischen Aufbau des Rücklaufverhinderers, was den steuerungstechnischen Aufbau der bioprozesstechnischen Anlage vereinfacht. Grundsätzlich kann es aber auch vorgesehen sein, dass der Rücklaufverhinderer eine elektrisch ansteuerbare Ventilanordnung aufweist, die auf ein Druckgefälle an den Anschlüssen des Rücklaufverhinderers schaltet, um den Rücklauf von flüssigem Medium bzw. Fermentationsbrühe zu verhindern.

Die Anordnung ist gemäß Anspruch 8 vorzugsweise so getroffen, dass die Zulaufleitung zwischen dem Pulvertransfersystem und dem Rücklaufverhinderer ständig frei von flüssigem Medium bzw. Fermentationsbrühe ist. Dies setzt eine entsprechende Dichtigkeit des Rücklaufverhinderers voraus. Zusätzlich kann es gemäß Anspruch 8 vorgesehen sein, dass die Zulaufleitung zwischen dem Rücklaufverhinderer und der Zulaufstelle zumindest bei einem stationären Hauptstrom frei von flüssigem Medium bzw. Fermentationsbrühe ist, jedenfalls in einem an den Rücklaufverhinderer angrenzenden Leitungsabschnitt der Zulaufleitung. Das ist insbesondere der Fall, wenn der bezogen auf den Rücklaufverhinderer zulaufstellenseitige Abschnitt der Zulaufleitung eine gewisse Mindestlänge aufweist, sodass in diesem Abschnitt der Zulaufleitung das flüssige Medium bzw. die Fermentationsbrühe nicht bis zum Rücklaufverhinderer steigen kann. Der Begriff "stationärer Hauptstrom" steht für den Zustand, auf den sich der Hauptstrom nach einer Änderung der Pumpleistung der Hauptstrompumpe, insbesondere nach dem Einschalten der Hauptstrompumpe, eingestellt hat.

Wie weiter oben angesprochen geht das Suspendieren oder Lösen des Zusatzes bzw. Filterhilfsmittels in dem flüssigen Medium bzw. in der Fermentationsbrühe zumindest zum Teil auf die Flüssigkeitsströmung bzw. die zwangsläufigen Verwirbelungen der Flüssigkeitsströmung in der Hauptleitung zurück. Bei der besonders bevorzugten Ausgestaltung gemäß Anspruch 9 ist es in einer Alternative zusätzlich so, dass das Suspendieren oder Lösen des Zusatzes bzw. Filterhilfsmittels in dem flüssigen Medium bzw. in der Fermentationsbrühe auch auf die Flüssigkeitsströmung bzw. Verwirbelungen in der Hauptstrompumpe zurückgeht.

Um das Suspendieren oder Lösen des Zusatzes bzw. Filterhilfsmittels in dem flüssigen Medium bzw. in der Fermentationsbrühe in der Hauptleitung zu unterstützen, ist es in einer bevorzugten Variante gemäß Anspruch 9 vorgesehen, das Innere der Hauptleitung mit Mischausformungen in Form von Strömungsleitprofilen, Strömungsbrechern oder dergleichen auszustatten. Sofern die Hauptleitung als Single-Use-Komponente aus einem Kunststoffmaterial ausgestaltet ist, lässt sich dies mit einfachen fertigungstechnischen Mitteln umsetzen.

Je nach flüssigem Medium bzw. Fermentationsbrühe einerseits und Zusatz bzw. Filterhilfsmittel andererseits kann es sein, dass eine hinreichende Durchmischung von Zusatz bzw. Filterhilfsmittel und flüssigem Medium bzw. Fermentationsbrühe in dem kurzen Abschnitt der Hauptleitung zwischen der Zulaufstelle und dem an die Hauptleitung angeschlossenen Anlagenteil bzw. der Filteranordnung noch nicht stattgefunden hat. Hierfür ist es gemäß Anspruch 10 vorzugsweise vorgesehen, dass über eine ansteuerbare Ventilanordnung eine Rezirkulation des flüssigen Mediums bzw. der Fermentationsbrühe eingeleitet wird. Der Zweck der Rezirkulation ist, dass bei nicht vollständigem Suspendieren oder Lösen des Zusatzes bzw. Filterhilfsmittels durch die Rückführung in einen mit Rührwerk versehenen Flüssigkeitsvorlagebehälter, insbesondere in einen gerührten Bioreaktor, der Suspendier- oder Löseschritt zunächst zu Ende geführt werden kann, um dann die Flussrichtung umzuschalten und dadurch von Rezirkulation insbesondere auf Filtration umzuschalten. Unter anderem hierfür ist die bioprozesstechnische Anlage mit einer Steueranordnung gemäß Anspruch 11 ausgestattet, von der die Ventilanordnung entsprechend ansteuerbar ist.

Die weiter bevorzugte Ausgestaltung gemäß Anspruch 12 betrifft im Ansatz eine geregelte Ansteuerung des Pulvertransfersystems und/oder der die Rezirkulation betreffenden Ventilanordnung und/oder der Hauptstrompumpe in Abhängigkeit von den Sensorwerten einer Sensoranordnung, die eine Charakteristik des um den Zusatz angereicherten flüssigen Mediums bzw. der um das Filterhilfsmittel angereicherten Fermentationsbrühe ermittelt. Damit ist es beispielsweise möglich, die Rezirkulation in Abhängigkeit von der Konzentration des Zusatzes bzw. Filterhilfsmittels ein- bzw. abzuschalten.

Nach einer weiteren Lehre gemäß Anspruch 13, der eigenständige Bedeutung zukommt, wird ein Verfahren für den Betrieb der vorschlagsgemäßen, bioprozesstechnischen Anlage als solches beansprucht.

Wesentlich bei dem vorschlagsgemäßen Verfahren ist, dass mittels des Pulvertransfersystems der Zusatz bzw. das Filterhilfsmittel in die Zulaufleitung in Pulverform eingespeist wird und mittels eines druckbeaufschlagten Transportgases zu der Zulaufstelle getrieben wird und dass zumindest ein Teil des eingespeisten Zusatzes bzw. Filterhilfsmittels innerhalb der Hauptleitung stromabwärts der Zulaufstelle in dem flüssigen Medium bzw. in der Fermentationsbrühe suspendiert oder gelöst wird, bevor das mit dem Zusatz angereicherte flüssige Medium bzw. die mit dem Filterhilfsmittel angereicherte Fermentationsbrühe das an die Hauptleitung angeschlossene Anlagenteil bzw. die Filteranordnung durchläuft oder erreicht. Angesichts der Tatsache, dass das vorschlagsgemäße Verfahren die Betriebsweise der vorschlagsgemäßen bioprozesstechnischen Anlage betrifft, darf auf alle diesbezüglichen Ausführungen zu der erstgenannten Lehre verwiesen werden.

Die weiter bevorzugte Ausgestaltung gemäß Anspruch 14 betrifft eine spezielle Betriebsweise der Hauptstrompumpe, sodass ein ungewünschter Durchlass des Rücklaufverhinderers vermieden wird.

Nach einer weiteren Lehre gemäß Anspruch 15, der ebenfalls eigenständige Bedeutung zukommt, wird die Verwendung eines steril verpackten Transfersatzes aus vorkonfektionierten Anlagekomponenten für den Aufbau einer vorschlagsgemäßen, bioprozesstechnischen Anlage beansprucht.

Wesentlich nach dieser weiteren Lehre ist, dass der Transfersatz in der sterilen Verpackung mindestens die Zulaufleitung einschließlich des Rücklaufverhinderers aufweist. Für die Montage der bioprozesstechnischen Anlage wird der Transfersatz entpackt, also der Verpackung entnommen, und an die bioprozesstechnische Anlage im Übrigen angeschlossen. Für den Fall, dass der Transfersatz nur die Zulaufleitung einschließlich des Rücklaufverhinderers aufweist, beschränkt sich der Anschluss des Transfersatzes an das Pulvertransfersystem einerseits und an die Hauptleitung, dort an der Zulaufstelle, andererseits.

Mit der vorschlagsgemäßen Verwendung ist erkannt worden, dass sich die vorschlagsgemäße, bioprozesstechnische Anlage, insbesondere aufgrund des Rücklaufverhinderers, besonders gut für die Umsetzung des oben angesprochenen Single-Use-Konzepts eignet. Dies betrifft zumindest die Zulaufleitung einschließlich des Rücklaufverhinderers.

Bei der weiter bevorzugten Ausgestaltung gemäß Anspruch 16 kann das Single-Use-Konzept aber auch auf die Komponenten der Hauptleitung sowie auf den Flüssigkeitsvorlagebehälter, insbesondere Bioreaktor, oder den Bioprozessbeutel angewendet werden. Für den Fall, dass dem Transfersatz mehrere Anlagenkomponenten zugeordnet sind, umfasst die Vorkonfektionierung vorzugsweise die Verbindung der Anlagenkomponenten untereinander, sodass der Anschluss des Transfersatzes an die bioprozesstechnische Anlage im Übrigen vereinfacht wird.

Bei der besonders bevorzugten Variante gemäß Anspruch 17 sind die dem Transfersatz zugeordneten Anlagenkomponenten vorzugsweise als Single-Use-Komponenten ausgestaltet, die, wie oben erläutert, weiter vorzugsweise aus einem Kunststoffmaterial ausgelegt sind.

Im Übrigen darf hinsichtlich der vorschlagsgemäßen Verwendung auf alle Ausführungen zu der erstgenannten Lehre verwiesen werden.

Im Folgenden wird die Erfindung anhand einer lediglich ein Ausführungsbeispiel darstellenden Zeichnung näher erläutert. In der Zeichnung zeigt
- Fig. 1: eine vorschlagsgemäße, bioprozesstechnische Anlage in einer schematischen Darstellung,
- Fig. 2: einen steril verpackten Transfersatz zur vorschlagsgemäßen Verwendung für den Aufbau einer vorschlagsgemäßen, bioprozesstechnischen Anlage a) in einer ersten Ausführungsform und b) in einer zweiten Ausführungsform.

Es darf vorab darauf hingewiesen werden, dass in der Zeichnung nur die Komponenten der vorschlagsgemäßen, bioprozesstechnischen Anlage 1 gezeigt sind, die für die Erläuterung der Lehren erforderlich sind. Entsprechend ist für eine gute Übersichtlichkeit auf die Darstellung einer Mehrzahl zusätzlich vorgesehener Ventile, Sensoren oder dergleichen verzichtet worden.

Bei diesem Ausführungsbeispiel steht die Suspension von Filterhilfsmitteln im Focus. Dies ist jedoch nicht beschränkend zu verstehen. Grundsätzlich ist stattdessen auch die Zugabe anderer Zusätze 47, insbesondere im Fall der Puffer- oder Medienherstellung ein Salz und/oder Nährstoff, ein denkbarer Anwendungsfall.

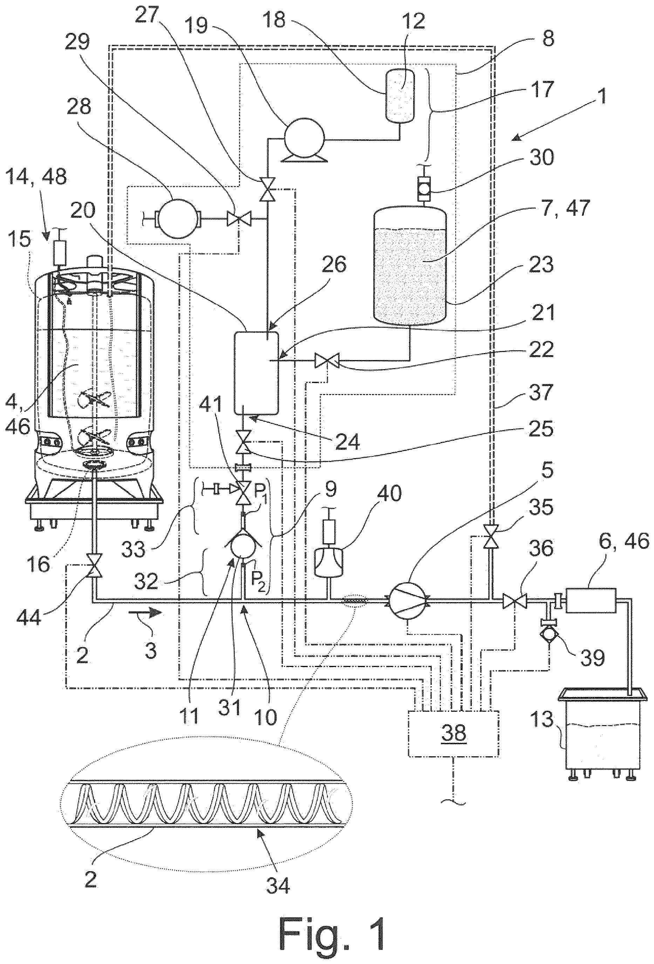

Die in Fig. 1 gezeigte bioprozesstechnische Anlage 1 ist mit einer Hauptleitung 2 zum Leiten eines Hauptstroms 3 aus Fermentationsbrühe 4, mit einer Hauptstrompumpe 5 zur Erzeugung des Hauptstroms 3 und mit einer an die Hauptleitung 2 angeschlossenen Filteranordnung 6 zur Filterung der Fermentationsbrühe 4 ausgestattet.

Anstelle von Fermentationsbrühe 4 kann in einem anderen Anwendungsfall auch ein anderes flüssiges Medium 45 vorgesehen sein.

Weiter kann anstelle einer Filteranordnung 6 in einem anderen Anwendungsfall auch ein anderes an die Hauptleitung 2 angeschlossenes Anlagenteil 46 vorgesehen sein, das mit dem flüssigen Medium beaufschlagbar ist, beispielsweise derart, dass es von dem flüssigen Medium durchströmbar ist oder dass das flüssige Medium in dieses einströmen kann.

Die Filteranordnung 6 arbeitet nach dem Prinzip der Anschwemmfiltration. Dadurch ist als Zusatz ein Filterhilfsmittel 7 in der Fermentationsbrühe 4 zu suspendieren, bevor die mit dem Filterhilfsmittel 7 angereicherte Fermentationsbrühe 4 die Filteranordnung 6 durchläuft.

Wesentlich ist nun, dass die bioprozesstechnische Anlage 1 hierfür ein Pulvertransfersystem 8 zur, insbesondere staubfreien, dosierten Abgabe des Filterhilfsmittels 7 und eine an das Pulvertransfersystem 8 angeschlossene Zulaufleitung 9 aufweist, die an einer Zulaufstelle 10 in die Hauptleitung 2 mündet. Fig. 1 zeigt, dass die Zulaufleitung 9 einen Rücklaufverhinderer 11 zum Verhindern eines Rücklaufs von Fermentationsbrühe 4 aufweist. Damit ist sichergestellt, dass die Zulaufleitung 9 zwischen dem Pulvertransfersystem 8 und dem Rücklaufverhinderer 11 frei von Fermentationsbrühe 4 ist. Dies ist ein wichtiger Aspekt für die Umsetzung des obigen Single-Use-Konzepts, wie noch erläutert wird.

Wesentlich ist weiter, dass das Pulvertransfersystem 8 das Filterhilfsmittel 7 in die Zulaufleitung 9 in Pulverform einspeist und mittels eines druckbeaufschlagten Transportgases 12 zu der Zulaufstelle 10 und insbesondere über die Zulaufstelle 10 hinaus in die Hauptleitung 2 treibt. Es findet also eine Strömung aus Transportgas 12 und Filterhilfsmittel 7 vom Pulvertransfersystem 8 zu der Zulaufstelle 10 statt, die dafür sorgt, dass das Filterhilfsmittel 7 in die von der Fermentationsbrühe 4 durchströmte Hauptleitung 2 eintritt. Als Folge davon wird zumindest ein Teil des eingespeisten Filterhilfsmittels 7 innerhalb der Hauptleitung 2 stromabwärts der Zulaufstelle 10 in der Fermentationsbrühe 4 suspendiert, bevor die mit dem Filterhilfsmittel 7 angereicherte Fermentationsbrühe 4 die Filteranordnung 6 durchläuft.

Nach dem Durchlaufen der Filteranordnung 6 wird die gefilterte Fermentationsbrühe 4 je nach Anwendungsbereich weiterverarbeitet, entsorgt oder zurückgeführt. In Fig. 1 ist im Sinne einer einfachen Darstellung lediglich ein Auffangbehälter 13 für die gefilterte Fermentationsbrühe gezeigt, was nicht beschränkend zu verstehen ist.

Ein Blick auf die Darstellung gemäß Fig. 1 zeigt, dass die Zugabe des Filterhilfsmittels 7 nur über die Zulaufleitung 9 und damit ohne irgendwelche Mischkammern vorgesehen ist, sodass sich eine Änderung der Dosierung des Filterhilfsmittels 7 durch das Pulvertransfersystem 8 nahezu unmittelbar auf die Konzentration des Filterhilfsmittels 7 in der Fermentationsbrühe 4 auswirkt. Die Reaktionszeit der bioprozesstechnischen Anlage 1 auf eine Änderung der Dosierung des Filterhilfsmittels 7 ist damit vorteilhaft kurz.

In bevorzugter Ausgestaltung ist die bioprozesstechnische Anlage 1 mit einem Bioreaktor 14 ausgestattet, der die Fermentationsbrühe 4 erzeugt und der in weiter bevorzugter Ausgestaltung einen zugeordneten Bioprozessbeutel 15 zur Aufnahme der Fermentationsbrühe 4 aufweist. Der Bioreaktor 14 ist mit seinem Drain-Port 16 an die Hauptleitung 2 angeschlossen. Aus dem Bioreaktor (14) wird die Fermentationsbrühe 4 über die Hauptleitung 2 in die Zulaufstelle 10 eingespeist.

Anstelle von einem Bioreaktor 14 kann in einem anderen Anwendungsfall auch ein anderer Flüssigkeitsvorlagebehälter 48 vorgesehen sein.

Im Sinne der Umsetzung des obigen Single-Use-Konzepts ist es vorzugsweise so, dass die Zulaufleitung 9 und/oder die Hauptleitung 2 und/oder der Rücklaufverhinderer 11 und/oder die Hauptstrompumpe 5 als Single-Use-Komponente bzw. als Single-Use-Komponenten ausgestaltet ist bzw. sind. In besonders bevorzugter Ausgestaltung ist mindestens eine dieser Komponenten zumindest zum Teil, vorzugsweise überwiegend, aus einem Kunststoffmaterial, vorzugsweise aus einem Silikonmaterial und/oder aus einem Polymermaterial, insbesondere PE (Polyethylen), PP (Polypropylen), PTFE (Polytetrafluorethylen), PBT (Polybutylenterephthalat), PSU (Polysulfon), PESU (Polyethersulfon), PC (Polycarbonat), ausgelegt.

Die Verwendung von Transfersets aus solchen Single-Use-Komponenten für den Aufbau der vorschlagsgemäßen, bioprozesstechnischen Anlage 1 ist Gegenstand einer weiteren Lehre, die weiter unten diskutiert wird.

Für die Auslegung des Pulvertransfersystems 8 sind verschiedene vorteilhafte Varianten denkbar. Vorzugsweise weist das Pulvertransfersystem 8 eine Transportgasquelle 17 für das druckbeaufschlagte Transportgas 12 auf, wobei das Pulvertransfersystem 8 das Filterhilfsmittel 7 mittels des druckbeaufschlagten Transportgases 12 in die Zulaufleitung 9 einspeist. Im Einzelnen weist die Transportgasquelle 17 hier und vorzugsweise ein Transportgasreservoir 18 und einen Kompressor 19 auf. Der Kompressor 19 pumpt das Transportgas 12 zur Zugabe des Filterhilfsmittels 7 derart durch eine mit Filterhilfsmittel 7 gefüllte Pulverkammer 20, dass das Filterhilfsmittel 7, wie oben angesprochen, mittels des druckbeaufschlagten Transportgases 12 in die Zulaufleitung 9 eingespeist wird. Bei dem Transportgas 12 handelt es sich vorzugsweise um ein Inertgas, beispielsweise um Stickstoff. Grundsätzlich kann es sich bei dem Transportgas 12 aber auch einfach um Luft handeln.

Die Pulverkammer 20 weist einen Pulvereingang 21 auf, der über ein dem Pulvereingang 21 zugeordnetes, ansteuerbares Ventil 22 an ein Pulverreservoir 23 angeschlossen ist. Die Pulverkammer 20 weist ferner einen Pulverausgang 24 auf, dem ein ansteuerbares Ventil 25 zugeordnet ist, dass wiederum an die Zulaufleitung 9 angeschlossen ist. Schließlich weist die Pulverkammer 20 einen Gasanschluss 26 auf, auf den der Kompressor 19 über ein dem Kompressor 19 zugeordnetes, ansteuerbares Ventil 27 aufgeschaltet werden kann. Zusätzlich wird auf den Anschluss 26 eine Vakuumpumpe 28 über ein der Vakuumpumpe 28 zugeordnetes, ansteuerbares Ventil 29 aufgeschaltet.

Zum Befüllen der Pulverkammer 20 sind das dem Pulverausgang 24 zugeordnete Ventil 25 und das dem Kompressor 19 zugeordnete Ventil 27 geschlossen, während das der Vakuumpumpe 28 zugeordnete Ventil 29 und das dem Pulvereingang 21 zugeordnete Ventil 22 geöffnet ist. In diesem Zustand führt der Betrieb der Vakuumpumpe 28 zu einem Absaugen von Filterhilfsmittel 7 aus dem Pulverreservoir 23 über das dem Pulvereingang 21 zugeordnete Ventil 22. Dabei wird der Einfachheit halber angenommen, dass angesichts des großen Volumens des Pulverreservoirs 23 zunächst kein Gas in das Pulverreservoir 23 nachströmen muss.

Nach der Befüllung der Pulverkammer 20 wird das der Vakuumpumpe 28 zugeordnete Ventil 29 geschlossen, während das dem Pulverausgang 24 zugeordnete Ventil 25 und das dem Kompressor 19 zugeordnete Ventil 27 geöffnet werden. Die anschließende Ansteuerung des Kompressors 19 führt zu einem Durchströmen der Pulverkammer 20 mit Transportgas 12 aus dem Transportgasreservoir 18, wodurch das in der Pulverkammer 20 nunmehr vorhandene Filterhilfsmittel 7, wie oben angesprochen, in die Zulaufleitung 9 eingespeist wird und, getrieben durch das druckbeaufschlagte Transportgas 12, die Zulaufstelle 10 erreicht.

Der oben beschriebene Vorgang kann wiederholt werden, bis die jeweils vorgesehene Menge an Filterhilfsmittel 7 in die Hauptleitung 2 eingespeist worden ist.

Ganz allgemein ist es hier und vorzugsweise so, dass das Filterhilfsmittel 7 mittels des Pulvertransfersystems 8 diskontinuierlich in die Zulaufleitung 9 und damit in die Hauptleitung 2 eingespeist wird. Dies lässt sich mit dem in der Zeichnung dargestellten, einfachen Aufbau leicht umsetzen. Alternativ kann es vorgesehen sein, dass das Pulvertransfersystem 8 das Filterhilfsmittel 7 nahezu kontinuierlich in die Zulaufleitung 9 einspeist.

Es wurde schon darauf hingewiesen, dass dem Rücklaufverhinderer 11 der Zulaufleitung 9 bei der vorschlagsgemäßen Lösung eine besondere Bedeutung zukommt. Der Rücklaufverhinderer 11 sperrt einen Fluidstrom in Richtung des Pulvertransfersystems 8. Bei Überschreiten eines Schalt-Druckgefälles in Durchlassrichtung des Rücklaufverhinderers 11 lässt der Rücklaufverhinderer 11 einen Filterhilfsmittelstrom in Richtung der Hauptleitung 2 durch. Das Druckgefälle ergibt sich aus der Differenz zwischen dem in Fig. 1 oberhalb des Rücklaufverhinderers 11 in der Zulaufleitung 9 herrschenden Druck P₁ und dem unterhalb des Rücklaufverhinderers 11 in der Zulaufleitung 9 herrschenden Druck P₂. Dabei fällt der Rücklaufverhinderer 11 erst in den Durchlasszustand, wenn das Druckgefälle in Durchlassrichtung das vorbestimmte Schalt-Druckgefälle erreicht. Je nach Auslegung des Rücklaufverhinderers 11 kann ein Schalt-Druckgefälle unterschiedlicher Größenordnung vorliegen.

Eine besonders einfache Ausgestaltung des Rücklaufverhinderers 11 ergibt sich dadurch, dass der Rücklaufverhinderer 11 nach Art eines Rückschlagventils aufgebaut ist und einen federelastischen oder mittels einer Federanordnung federvorgespannten Ventilkörper 31 aufweist.

In der erstgenannten Variante kann der Rücklaufverhinderer 11 beispielsweise als Lippenventil ausgestaltet sein, bei dem sich je nach Flussrichtung in den Ventilkörper 31 eingeformte Lippen öffnen oder schließen. Bei der Ausgestaltung des Rücklaufverhinderers 11 mit einem federvorgespannten Ventilkörper kann der Ventilkörper beispielsweise als Rückschlagklappe, als Ball oder dergleichen ausgestaltet sein.

In besonders bevorzugter Ausgestaltung ist der Rücklaufverhinderer 11 so ausgestaltet, dass die Zulaufleitung 9 zwischen dem Pulvertransfersystem 8 und dem Rücklaufverhinderer 11 ständig frei von Fermentationsbrühe 4 ist. Zusätzlich ist es vorzugsweise so, dass die Zulaufleitung 9 zwischen dem Rücklaufverhinderer 11 und der Zulaufstelle 10 zumindest bei einem stationären Hauptstrom 3 frei von Fermentationsbrühe 4 ist. Hier zeigt sich, dass der in Fig. 1 untere Abschnitt 32 der Zulaufleitung 9, also der Abschnitt zwischen dem Rücklaufverhinderer 11 und der Zulaufstelle 10, vorzugsweise eine gewisse Mindestlänge aufweisen kann, um sicher zu verhindern, dass Fermentationbrühe 4 bei einem nicht vollständigen Schließen des Rücklaufverhinderers 11 über den Rücklaufverhinderer 11 in den in Fig. 1 oberen Abschnitt 33 der Zulaufleitung, also in den Abschnitt 33 zwischen dem Rücklaufverhinderer 11 und dem Pulvertransfersystem 8, gelangt.

Dennoch darf darauf hingewiesen werden, dass die Zulaufleitung 9 grundsätzlich auch ohne den unteren Abschnitt 32 und/oder ohne den oberen Abschnitt 33 auskommt. Im Extremfall kann es sogar vorgesehen sein, dass die Zulaufleitung 9 ausschließlich von dem Rücklaufverhinderer 11 bereitgestellt wird.

Das Suspendieren des Filterhilfsmittels 7 in der Fermentationsbrühe 4 geht hier und vorzugsweise auf die Flüssigkeitsströmung in der Hauptleitung 2 und/oder in der Hauptstrompumpe 5 zurück. Insbesondere die in der Hauptstrompumpe 5 entstehenden Strömungsturbulenzen führen zu einer besonders guten Durchmischung des Filterhilfsmittels 7 mit der Fermentationsbrühe 4, sodass die Strecke zwischen der Zulaufstelle 10 und der Filteranordnung 6 für eine hinreichende Vermischung sorgen kann. Um die Vermischung in der Hauptleitung 2 weiter zu unterstützen, ist es vorzugsweise vorgesehen, dass die Hauptleitung 2, insbesondere stromabwärts der Zulaufstelle 10, mindestens einen Mischabschnitt aufweist, wobei die Hauptleitung 2 im Mischabschnitt Mischausformungen 34 in Form von Stömungsleitprofilen, Strömungsbrechern oder dergleichen aufweist. In dem in Fig. 1 dargestellten Ausführungsbeispiel ist dies durch helixartige Mischausformungen 34, insbesondere in der Art eines statischen Mischers wie er z.B. bei Zwei-Komponenten-Mischern bekannt ist, umgesetzt worden. Diese Mischausformungen 34 können beispielsweise als in die Hauptleitung 2 eingesetzte Kunststoffhelix realisiert sein, was herstellungstechnisch einfach umzusetzen ist. Dabei kann die Mischausformung sowohl stromauf- als auch stromabwärts der Hauptstrompumpe 5 angeordnet sein.

Alternativ oder zusätzlich kann es vorgesehen sein, dass die Hauptleitung 2 eingangsseitig der Filteranordnung 6 eine ansteuerbare Ventilanordnung 35, 36 aufweist, über die die Fermentationsbrühe 4, hier und vorzugsweise zurück in den Bioreaktor 14, rezirkulierbar ist. Eine entsprechende Rezirkulationsleitung 37, die in den Bioreaktor 14 zurückgeführt ist, zeigt Fig. 1 in gestrichelter Linie. Die Rezirkulation wird dadurch ausgelöst, dass das Ventil 35 geöffnet, und das Ventil 36 geschlossen wird.

Die dargestellte, bioprozesstechnische Anlage 1 weist hier und vorzugsweise eine Steueranordnung 38 auf, wobei das Pulvertransfersystem 8 und/oder die die Rezirkulation betreffende Ventilanordnung 35, 36 und/oder die Hauptstrompumpe 5 von der Steueranordnung 38 ansteuerbar ist bzw. sind. Bei der Steueranordnung 38 handelt es sich vorzugsweise um eine elektronische Steueranordnung, auf der weiter vorzugsweise eine Steuersoftware läuft.

Mit der Steueranordnung 38 lässt sich der Betrieb der vorschlagsgemäßen, bioprozesstechnischen Anlage 1 in weiten Bereichen automatisieren. Dies betrifft insbesondere die Automatisierung der Zugabe des Filterhilfsmittels 7. Hierfür ist vorzugsweise eine Sensoranordnung 39 vorgesehen, die vorzugsweise eine Charakteristik der um das Filterhilfsmittel 7 angereicherten Fermentationsbrühe 4 ermittelt. Dies könnte beispielsweise eine Konzentrations-, Trübungs- oder Viskositätsmessung sein. Eine solche Sensoranordnung 39 ist in Fig. 1 der Filteranordnung 6 unmittelbar vorgeschaltet. Die Sensoranordnung 39 kann grundsätzlich eine Mehrzahl in der bioprozesstechnischen Anlage 1 verteilter Sensoren umfassen.

Vorzugsweise ist es so, dass die Steueranordnung 38 das Pulvertransfersystem 8 und/oder die die Rezirkulation betreffende Ventilanordnung 35, 36 und/oder die Hauptstrompumpe 5 in Abhängigkeit von den Sensorwerten der Sensoranordnung 39 ansteuert. In besonders bevorzugter Ausgestaltung handelt es sich bei der ermittelten Charakteristik um die Konzentration des Filterhilfsmittels 7 in der Fermentationsbrühe 4 und/oder um den zeitlichen Verlauf der Konzentration des Filterhilfsmittels 7 in der Fermentationsbrühe 4. Sinkt beispielsweise die Konzentration des Filterhilfsmittels 7 in der Fermentationsbrühe 4 unter einen vorbestimmten Grenzwert, so steuert die Steueranordnung 38 das Pulvertransfersystem 8 an, zusätzliches Filterhilfsmittel 7 zuzugeben. Alternativ kann der Eingangsdruck bzw. Druckverlauf, insbesondere in der Hauptleitung 2, im Sinne der obigen Charakteristik als Steuerungsgröße eingesetzt werden. Damit kann die Menge an Filterhilfsmittel dynamisch an den Druckverlauf angepasst werden gemäß einer dynamischen Anschwemmfiltration. Dies erfolgt durch eine entsprechende Ansteuerung der Ventile 25, 27 und 29, wie weiter oben erläutert worden ist. Eine weitere Steuerungsgröße zur Filterhilfsmitteldosierung ist die Wägung des Filterhilfsmittels im Vorlagebehälter.

Alternativ oder zusätzlich kann es vorgesehen sein, dass die Sensoranordnung 39 den Volumenstrom der Fermentationsbrühe 4 durch die Hauptleitung 2 erfasst und die Zugabe des Filterhilfsmittels 7 in Abhängigkeit von dem ermittelten Volumenstrom steuert.

Es wurde weiter oben erläutert, dass die vorschlagsgemäße Zugabe des Filterhilfsmittels 7 mittels eines druckbeaufschlagten Transportgases 12 erfolgt. Je nach Auslegung des Leitungssystems der bioprozesstechnischen Anlage 1 kann es erforderlich sein, ein Ausgleichssystem 40 vorzusehen, um die Energie des in das Leitungssystem eingebrachten Transportgases 12 zu absorbieren, ohne das Leitungssystem mechanisch übermäßig zu belasten.

Bei dem Ausgleichssystem 40 kann es sich beispielsweise um einen einfachen Ausgleichsbehälter handeln, wie in Fig. 1 angedeutet ist. Im Einzelnen weist das in Fig. 1 gezeigte Ausgleichssystem 40 neben dem Ausgleichsbehälter einen Entlüftungsfilter auf, über den das Transportgas 12 aus dem System abgeleitet wird. Dadurch wird vermieden, dass das Transportgas 12 die Anschwemmfiltration und die Ausbildung eines geeigneten Filterkuchens negativ beeinflusst. Der Ausgleichsbehälter wirkt gewissermaßen als "Gasfalle".

Bei dem Ausgleichsbehälter des Ausgleichssystems 40 handelt es sich vorzugsweise um einen Rührbeutel, um die suspendierten Partikel des Filterhilfsmittels 7 in Schwebe zu halten.

Alternativ kann es auch vorgesehen sein, dass beim vorschlagsgemäßen Zugeben von Filterhilfsmittel 7 mittels des Pulvertransfersystems 8 die Rezirkulationsleitung 37 mittels der Ventilanordnung 35, 36 stets aktiviert wird, sodass das Transportgas 12 in den Bioreaktor 14 gelangt und dort über den Exhaust-Port abgeblasen wird.

Es darf noch darauf hingewiesen werden, dass am unteren Auslauf des Bioreaktors 14 ein weiteres, ansteuerbares Ventil 44 vorgesehen ist, das im statischen Zustand dafür sorgt, dass der hydrostatische Druck der Fermentationsbrühe 4 im Bioreaktor 14 nicht an der Hauptleitung 2 ansteht.

Nach einer weiteren Lehre, der eigenständige Bedeutung zukommt, wird ein Verfahren für den Betrieb einer vorschlagsgemäßen, bioprozesstechnischen Anlage 1 als solches beansprucht.

Nach dem vorschlagsgemäßen Verfahren ist es so, dass mittels des Pulvertransfersystems 8 das Filterhilfsmittel 7 in die Zulaufleitung 9 in Pulverform eingespeist wird und mittels eines druckbeaufschlagten Transportgases 12 zu der Zulaufstelle 10 getrieben wird. Dabei wird zumindest ein Teil des eingespeisten Filterhilfsmittels 7 innerhalb der Hauptleitung 2 stromabwärts der Zulaufstelle 10 in der Fermentationsbrühe 4 suspendiert, bevor die mit dem Filterhilfsmittel 7 angereicherte Fermentationsbrühe 4 die Filteranordnung 6 durchläuft. Auf alle obigen Ausführungen zu der Betriebsweise der vorschlagsgemäßen, bioprozesstechnischen Anlage 1 darf verwiesen werden.

Das vorschlagsgemäße Verfahren betrifft insbesondere die Ansteuerung von Komponenten wie das Pulvertransfersystem 8, die Hauptstrompumpe 5 oder dergleichen. Im Einzelnen sind die oben angesprochenen Ansteuerungsvarianten des Pulvertransfersystems 8 mittels der Steueranordnung 38 Bestandteil des vorschlagsgemäßen Verfahrens.

Bevor auf die konkrete Umsetzung des Single-Use-Konzepts eingegangen wird, darf noch auf eine bevorzugte Maßnahme hingewiesen werden, die die Mehrfachnutzung des Pulvertransfersystems 8 betrifft. Bei dem in Fig. 1 dargestellten und insoweit bevorzugten Ausführungsbeispiel ist es nämlich so, dass die Zulaufleitung 9 im oberen Abschnitt 33, also zwischen dem Rücklaufverhinderer 11 und dem Pulvertransfersystem 8, ein Steam-To-Ventil 41 (STV) aufweist. Ganz allgemein verbindet ein Steam-To-Ventil eine Einweg-Komponente, vorzugsweise die Zulaufleitung 9, mit einer Mehrweg-Komponente, vorzugsweise dem Pulvertransfersystem 8, wobei die Mehrwegkomponente über das Steam-To-Ventil vor Prozeßbeginn mit Dampf sterilisiert werden kann, um nachfolgend eine Verbindung zu der Einweg-Komponente, die weiter vorzugsweise gamma-sterilisiert bereitgestellt wird, herzustellen.

Vorliegend ist es also so, dass das Steam-To-Ventil 41 vor und/oder nach der Zugabe von Filterhilfsmittel 7 eine Dampfsterilisation des Anschlusses des Pulvertransfersystems 8 erlaubt. Dadurch wird das Kontaminationsrisiko auch nach einem Austausch der Zulaufleitung 9 weiter reduziert.

Nach einer weiteren Lehre, der eigenständige Bedeutung zukommt, wird eine Verwendung eines steril verpackten Transfersatzes 42 aus vorkonfektionierten Anlagenkomponenten für den Aufbau einer vorschlagsgemäßen, bioprozesstechnischen Anlage 1 als solche beansprucht.

Nach dem Entpacken des Transfersatzes 42 aus der Verpackung 43 wird der Transfersatz an die bioprozesstechnische Anlage 1 im Übrigen angeschlossen.

Alternativ kann der Transfersatz 42 aus mehreren Einzelkomponenten-Sets bestehen, die mit Hilfe von Sterilkonnektoren beim Aufbau miteinander verbunden werden. Dann ist den Einzelkomponenten-Sets jeweils eine separate sterile Teilverpackung zugeordnet.

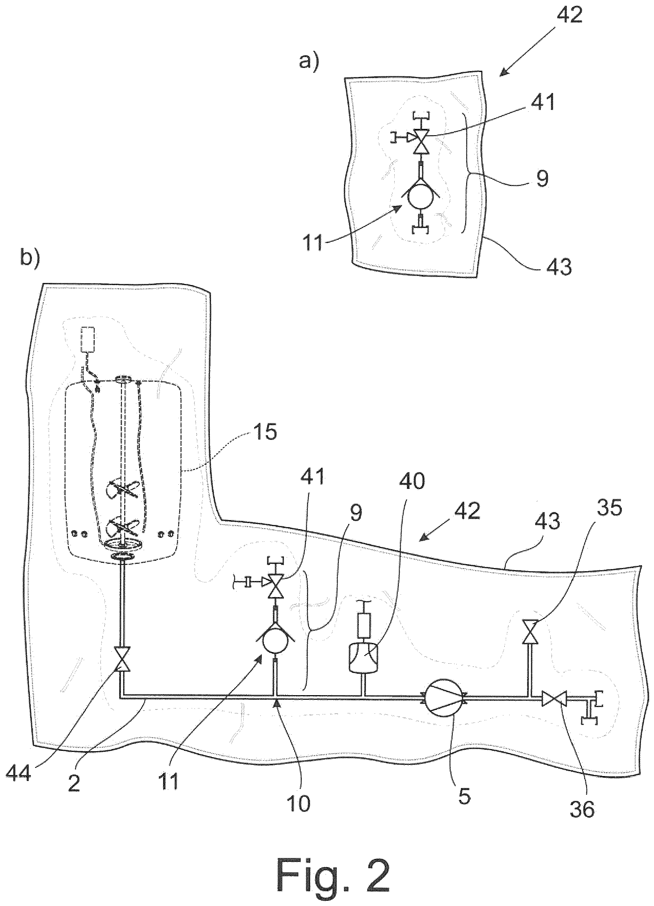

Zwei bevorzugte Varianten für einen zu verwendenden Transfersatz 42 sind der Darstellung gemäß Fig. 2 zu entnehmen. Nach der vorschlagsgemäßen Verwendung weist der Transfersatz 42 in der sterilen Verpackung 43 mindestens die Zulaufleitung 9 einschließlich des Rücklaufverhinderers 11 auf. Bei dem in Fig. 2a gezeigten und insoweit bevorzugten Ausführungsbeispiel ist der Zulaufleitung 9 zusätzlich das oben angesprochene Steam-To-Ventil 41 zugeordnet.

Zusätzlich kann es vorgesehen sein, dass der Transfersatz 42 in der sterilen Verpackung 43 zusätzlich die Hauptleitung 2, hier und vorzugsweise einschließlich der Hauptstrompumpe 5, aufweist, die vorkonfektioniert an der Zulaufstelle 10 an die Zulaufleitung 9 angeschlossen ist. Weiter vorzugsweise kann es vorgesehen sein, dass der Transfersatz 42 in der sterilen Verpackung 43 zumindest einen Teil des Bioreaktors 14, hier und vorzugsweise einen dem Bioreaktor 14 zugeordneten Bioprozessbeutel 15, aufweist, der vorkonfektioniert an der Zulaufstelle 10 an die Hauptleitung 2 angeschlossen ist.

In besonders bevorzugter Ausgestaltung handelt es sich bei den dem Transfersatz 42 zugeordneten Anlagenkomponenten um Single-Use-Komponenten. Dies betrifft die Zulaufleitung 9 und/oder die Hauptleitung 2 und/oder den Rücklaufverhinderer 11 und/oder die Hauptstrompumpe 5. In besonders bevorzugter Ausgestaltung sind die dem Transfersatz 42 zugeordneten Komponenten zumindest zum Teil, vorzugsweise überwiegend, aus einem, vorzugsweise flexiblen, Kunststoffmaterial, vorzugsweise aus einem Silikonmaterial und/oder aus einem Polymermaterial, insbesondere PE (Polyethylen), PP (Polypropylen), PTFE (Polytetrafluorethylen), PBT (Polybutylenterephthalat), PSU (Polysulfon), PESU (Polyethersulfon), PC (Polycarbonat), ausgelegt. Auch insoweit darf auf die Ausführungen zu der vorschlagsgemäßen, bioprozesstechnischen Anlage 1 verwiesen werden.

## Patentansprüche

1. Bioprozesstechnische Anlage mit einer Hauptleitung (2) zum Leiten eines Hauptstroms (3) aus einem flüssigen, insbesondere biologischen, Medium (45), mit einer Hauptstrompumpe (5) zur Erzeugung des Hauptstroms (3) und mit einem an die Hauptleitung (2) angeschlossenen Anlagenteil (46),
**dadurch gekennzeichnet,**
**dass** die bioprozesstechnische Anlage (1) ein Pulvertransfersystem (8) zur, insbesondere staubfreien, dosierten Abgabe eines Zusatzes (47) und eine an das Pulvertransfersystem (8) angeschlossene Zulaufleitung (9) aufweist, die an einer Zulaufstelle (10) in die Hauptleitung (2) mündet und die einen Rücklaufverhinderer (11) zum Verhindern eines Rücklaufs von flüssigem Medium (45) aufweist, dass das Pulvertransfersystem (8) den Zusatz (47) in die Zulaufleitung (9) in Pulverform einspeist und mittels eines druckbeaufschlagten Transportgases (12) zu der Zulaufstelle (10) treibt und dass zumindest ein Teil des eingespeisten Zusatzes (47) innerhalb der Hauptleitung (2) stromabwärts der Zulaufstelle (10) in das flüssige Medium (45) eingegeben wird, bevor das mit dem Zusatz (47) angereicherte flüssige Medium (45) das an die Hauptleitung (2) angeschlossene Anlagenteil (46) durchläuft oder erreicht.

2. Bioprozesstechnische Anlage nach Anspruch 1, **dadurch gekennzeichnet, dass** das flüssige Medium (45) Fermentationsbrühe (4) ist, und/oder,
dass das an die Hauptleitung (2) angeschlossene Anlagenteil (46) ein bioprozesstechnisches Funktionselement, vorzugsweise eine Filteranordnung (6) zur Filterung der Fermentationsbrühe (4) oder ein Aufnahmebehälter zur Aufnahme des flüssigen Mediums (45), ist, und/oder,
dass der Zusatz (47) ein Filterhilfsmittel (7) und/oder Salz und/oder Nährstoff ist.

3. Bioprozesstechnische Anlage nach Anspruch 1 oder 2,
mit einer Hauptleitung (2) zum Leiten eines Hauptstroms (3) aus Fermentationsbrühe (4), mit einer Hauptstrompumpe (5) zur Erzeugung des Hauptstroms (3) und mit einer an die Hauptleitung (2) angeschlossenen Filteranordnung (6) zur Filterung der Fermentationsbrühe (4),
wobei die bioprozesstechnische Anlage (1) ein Pulvertransfersystem (8) zur, insbesondere staubfreien, dosierten Abgabe eines Filterhilfsmittels (7) und eine an das Pulvertransfersystem (8) angeschlossene Zulaufleitung (9) aufweist, die an einer Zulaufstelle (10) in die Hauptleitung (2) mündet und die einen Rücklaufverhinderer (11) zum Verhindern eines Rücklaufs von Fermentationsbrühe (4) aufweist, wobei das Pulvertransfersystem (8) das Filterhilfsmittel (7) in die Zulaufleitung (9) in Pulverform einspeist und mittels eines druckbeaufschlagten Transportgases (12) zu der Zulaufstelle (10) treibt und wobei zumindest ein Teil des eingespeisten Filterhilfsmittels (7) innerhalb der Hauptleitung (2) stromabwärts der Zulaufstelle (10) in der Fermentationsbrühe (4) suspendiert wird, bevor die mit dem Filterhilfsmittel (7) angereicherte Fermentationsbrühe (4) die Filteranordnung (6) durchläuft.

4. Bioprozesstechnische Anlage nach einem der vorhergehenden Ansprüche, **dadurch gekennzeichnet, dass** die bioprozesstechnische Anlage (1) einen Flüssigkeitsvorlagebehälter (48), insbesondere einen Bioreaktor (14), insbesondere einen Bioreaktor (14) mit einem zugeordneten Bioprozessbeutel (15), aufweist, und dass aus dem Flüssigkeitsvorlagebehälter (48) das flüssige Medium (45) über die Hauptleitung (2) in die Zulaufstelle (10) eingespeist wird.

5. Bioprozesstechnische Anlage nach einem der vorhergehenden Ansprüche, **dadurch gekennzeichnet, dass** die Zulaufleitung (9) und/oder die Hauptleitung (2) und/oder der Rücklaufverhinderer (11) und/oder die Hauptstrompumpe (5), insbesondere als Single-Use-Komponente, zumindest zum Teil aus einem Kunststoffmaterial ausgelegt ist, vorzugsweise aus einem Silikonmaterial und/oder PE (Polyethylen), PP (Polypropylen), PTFE (Polytetrafluorethylen), PBT (Polybutylenterephthalat), PSU (Polysulfon), PESU (Polyethersulfon), PC (Polycarbonat).

6. Bioprozesstechnische Anlage nach einem der vorhergehenden Ansprüche, **dadurch gekennzeichnet, dass** das Pulvertransfersystem (8) eine Transportgasquelle (17) für das druckbeaufschlagte Transportgas (12) aufweist und dass das Pulvertransfersystem (8) den Zusatz (47) mittels des druckbeaufschlagten Transportgases (12) in die Zulaufleitung (9) einspeist, vorzugsweise, dass das Pulvertransfersystem (8) den Zusatz (47) diskontinuierlich in die Zulaufleitung (9) einspeist, oder, dass das Pulvertransfersystem (8) den Zusatz (47) kontinuierlich in die Zulaufleitung (9) einspeist.

7. Bioprozesstechnische Anlage nach einem der vorhergehenden Ansprüche, **dadurch gekennzeichnet, dass** der Rücklaufverhinderer (11) einen Fluidstrom in Richtung des Pulvertransfersystems (8) sperrt und bei Überschreiten eines Schalt-Druckgefälles in Durchlassrichtung des Rücklaufverhinderers (11) einen Strom aus dem Zusatz, insbesondere einen Filterhilfsmittelstrom, in Richtung der Hauptleitung (2) durchlässt, und/oder, dass der Rücklaufverhinderer (11) nach Art eines Rückschlagventils aufgebaut ist und einen federelastischen oder mittels einer Federanordnung federvorgespannten Ventilkörper (31) aufweist.

8. Bioprozesstechnische Anlage nach einem der vorhergehenden Ansprüche, **dadurch gekennzeichnet, dass** die Zulaufleitung (9) zwischen dem Pulvertransfersystem (8) und dem Rücklaufverhinderer (11) ständig frei von flüssigem Medium (45) ist, vorzugsweise, dass die Zulaufleitung (9) zwischen dem Rücklaufverhinderer (11) und der Zulaufstelle (10) zumindest bei einem stationären Hauptstrom frei von flüssigem Medium (45) ist.

9. Bioprozesstechnische Anlage nach einem der vorhergehenden Ansprüche, **dadurch gekennzeichnet, dass** der Zusatz (47) in dem flüssigen Medium (45) suspendiert oder gelöst wird, vorzugsweise, dass das Suspendieren oder Lösen des Zusatzes (47) in dem flüssigen Medium (45) auf die Flüssigkeitsströmung in der Hauptleitung (2) und/oder in der Hauptstrompumpe zurückgeht, vorzugsweise, dass die Hauptleitung (2), insbesondere stromabwärts der Zulaufstelle (10), mindestens einen Mischabschnitt aufweist und dass die Hauptleitung (2) im Mischabschnitt Mischausformungen (34) in Form von Strömungsleitprofilen, Strömungsbrechern oder dergleichen aufweist.

10. Bioprozesstechnische Anlage nach einem der vorhergehenden Ansprüche, **dadurch gekennzeichnet, dass** die Hauptleitung (2) eingangsseitig des an die Hauptleitung (2) angeschlossenen Anlagenteils (46) eine ansteuerbare Ventilanordnung (35, 36) aufweist, über die das flüssige Medium (45), insbesondere zurück in den Flüssigkeitsvorlagebehälter (48), rezirkulierbar ist, vorzugsweise, dass die Ventilanordnung (35, 36) über Steuersignale ansteuerbar ist.

11. Bioprozesstechnische Anlage nach einem der vorhergehenden Ansprüche, **dadurch gekennzeichnet, dass** die bioprozesstechnische Anlage (1) eine Steueranordnung (38) aufweist und dass das Pulvertransfersystem (8) und/oder die die Rezirkulation betreffende Ventilanordnung (35, 36) und/oder die Hauptstrompumpe (5) von der Steueranordnung (38) ansteuerbar ist bzw. sind.

12. Bioprozesstechnische Anlage nach Anspruch 11, **dadurch gekennzeichnet, dass** eine Sensoranordnung (39) vorgesehen ist, die eine Charakteristik des um den Zusatz (47) angereicherten flüssigen Mediums (45) ermittelt und dass die Steueranordnung (38) das Pulvertransfersystem und/oder die die Rezirkulation betreffende Ventilanordnung (35, 36) und/oder die Hauptstrompumpe (5) in Abhängigkeit von den Sensorwerten der Sensoranordnung (39) ansteuert, vorzugsweise, dass die ermittelte Charakteristik die Konzentration des Zusatzes (47) in dem flüssigen Medium (45) und/oder der zeitliche Verlauf der Konzentration des Zusatzes (47) in dem flüssigen Medium (45) und/oder der Eingangsdruck bzw. Druckverlauf , insbesondere in der Hauptleitung (2), ist.

13. Verfahren für den Betrieb einer bioprozesstechnischen Anlage (1) nach einem der vorhergehenden Ansprüche,
**dadurch gekennzeichnet,**
**dass** mittels des Pulvertransfersystems (8) der Zusatz (47) in die Zulaufleitung (9) in Pulverform eingespeist wird und mittels eines druckbeaufschlagten Transportgases (12) zu der Zulaufstelle (10) getrieben wird und dass zumindest ein Teil des eingespeisten Zusatzes (47) innerhalb der Hauptleitung (2) stromabwärts der Zulaufstelle (10) in das flüssige Medium (45) gegeben wird, bevor das mit dem Zusatz (47) angereicherte flüssige Medium (45) das an die Hauptleitung (2) angeschlossene Anlagenteil (46) durchläuft oder erreicht.

14. Verfahren nach Anspruch 13, **dadurch gekennzeichnet, dass** die Hauptstrompumpe (5) so betrieben wird, dass das Schalt-Druckgefälle in Durchlassrichtung des Rücklaufverhinderers (11) stets unterschritten wird.

15. Verwendung eines steril verpackten Transfersatzes (42) aus vorkonfektionierten Anlagenkomponenten für den Aufbau einer bioprozesstechnischen Anlage (1) nach einem der Ansprüche 1 bis 12,
**dadurch gekennzeichnet,**
**dass** der Transfersatz in der sterilen Verpackung die Zulaufleitung einschließlich des Rücklaufverhinderers (11) aufweist.

16. Verwendung nach Anspruch 15, **dadurch gekennzeichnet, dass** der Transfersatz (42) in der sterilen Verpackung (43) zusätzlich die Hauptleitung (2), vorzugsweise einschließlich der Hauptstrompumpe (5), aufweist, die vorkonfektioniert an der Zulaufstelle (10) an die Zulaufleitung (9) angeschlossen ist, vorzugsweise, dass der Transfersatz (42) in der sterilen Verpackung (43) zumindest einen Teil des Flüssigkeitsvorlagebehälter (48), insbesondere des Bioreaktors (14), insbesondere einen dem Bioreaktor (14) zugeordneten Bioprozessbeutel (15), aufweist, der vorkonfektioniert an der Zulaufstelle (10) an die Hauptleitung (2) angeschlossen ist.

17. Verwendung nach Anspruch 15 oder 16, **dadurch gekennzeichnet, dass** die Zulaufleitung (9) und/oder die Hauptleitung (2) und/oder der Rücklaufverhinderer (11) und/oder die Hauptstrompumpe (5), insbesondere als Single-Use-Komponente, zumindest zum Teil aus einem Kunststoffmaterial ausgelegt ist bzw. sind, vorzugsweise aus einem Silikonmaterial und/oder PE (Polyethylen), PP (Polypropylen), PTFE (Polytetrafluorethylen), PBT (Polybutylenterephthalat) , PSU (Polysulfon), PESU (Polyethersulfon), PC (Polycarbonat).

## Claims

1. Bioprocessing system having a main line (2) for conduction of a main flow (3) of a liquid, in particular biological, medium (45), having a main flow pump (5) for generation of the main flow (3), and having a system part (46) connected to the main line (2),
**characterized**
**in that** the bioprocessing system (1) comprises a powder transfer system (8) for metered delivery, in particular dust-free metered delivery, of an additive (47) and a feed line (9) which is connected to the powder transfer system (8) and which opens into the main line (2) at a feed point (10) and which comprises a return flow preventer (11) for prevention of a return flow of liquid medium (45), in that the powder transfer system (8) feeds the additive (47) into the feed line (9) in powder form and drives it toward the feed point (10) by means of a pressurized transport gas (12), and in that at least some of the fed additive (47) is input into the liquid medium (45) within the main line (2) downstream of the feed point (10) before the liquid medium (45) enriched with the additive (47) passes through or reaches the system part (46) connected to the main line (2).

2. Bioprocessing system according to claim 1, **characterized**
**in that** the liquid medium (45) is fermentation broth (4), and/or
**in that** the system part (46) connected to the main line (2) is a bioprocessing functional element, preferably a filter arrangement (6) for filtration of the fermentation broth (4) or an accommodation container for accommodation of the liquid medium (45), and/or
**in that** the additive (47) is a filter aid (7) and/or salt and/or nutrient.

3. Bioprocessing system according to claim 1 or 2,
having a main line (2) for conduction of a main flow (3) of fermentation broth (4), having a main flow pump (5) for generation of the main flow (3), and having a filter arrangement (6) connected to the main line (2) for filtration of the fermentation broth (4),
wherein the bioprocessing system (1) comprises a powder transfer system (8) for metered delivery, in particular dust-free metered delivery, of a filter aid (7) and a feed line (9) which is connected to the powder transfer system (8) and which opens into the main line (2) at a feed point (10) and which comprises a return flow preventer (11) for prevention of a return flow of fermentation broth (4), wherein the powder transfer system (8) feeds the filter aid (7) into the feed line (9) in powder form and drives it toward the feed point (10) by means of a pressurized transport gas (12), and wherein at least some of the fed filter aid (7) is suspended in the fermentation broth (4) within the main line (2) downstream of the feed point (10) before the fermentation broth (4) enriched with the filter aid (7) passes through the filter arrangement (6).

4. Bioprocessing system according to any of the preceding claims, **characterized in that** the bioprocessing system (1) comprises a liquid storage container (48), in particular a bioreactor (14), in particular a bioreactor (14) having an assigned bioprocess bag (15), and **in that** the liquid medium (45) is fed from the liquid storage container (48) into the feed point (10) via the main line (2).

5. Bioprocessing system according to any of the preceding claims, **characterized in that** the feed line (9) and/or the main line (2) and/or the return flow preventer (11) and/or the main flow pump (5), in particular as single-use components, is/are formed at least in part from a plastics material, preferably from a silicone material and/or PE (polyethylene), PP (polypropylene), PTFE (polytetrafluoroethylene), PBT (polybutylene terephthalate), PSU (polysulfone), PESU (polyether sulfone), PC (polycarbonate).

6. Bioprocessing system according to any of the preceding claims, **characterized in that** the powder transfer system (8) comprises a transport gas source (17) for the pressurized transport gas (12), and **in that** the powder transfer system (8) feeds the additive (47) into the feed line (9) by means of the pressurized transport gas (12), preferably **in that** the powder transfer system (8) feeds the additive (47) discontinuously into the feed line (9) or **in that** the powder transfer system (8) feeds the additive (47) continuously into the feed line (9).

7. Bioprocessing system according to any of the preceding claims, **characterized in that** the return flow preventer (11) blocks fluid flow in the direction of the powder transfer system (8) and, upon exceeding of a switching pressure gradient in the flow direction of the return flow preventer (11), lets through a flow of the additive, in particular a flow of filter aid, in the direction of the main line (2), and/or **in that** the return flow preventer (11) is constructed in the manner of a check valve and comprises a valve body (31) which is spring-elastic or which is spring-preloaded by means of a spring arrangement.

8. Bioprocessing system according to any of the preceding claims, **characterized in that** the feed line (9) is always free of liquid medium (45) between the powder transfer system (8) and the return flow preventer (11), preferably **in that** the feed line (9) is free of liquid medium (45) between the return flow preventer (11) and the feed point (10) at least in the case of a steady main flow.

9. Bioprocessing system according to any of the preceding claims, **characterized in that** the additive (47) is suspended or dissolved in the liquid medium (45), preferably **in that** the suspension or dissolution of the additive (47) in the liquid medium (45) stems from the liquid flow in the main line (2) and/or in the main flow pump, preferably **in that** the main line (2) comprises at least one mixing section, in particular downstream of the feed point (10), and **in that** the main line (2) comprises mixing shapes (34) in the form of flow guiding profiles, baffles or the like in the mixing section.

10. Bioprocessing system according to any of the preceding claims, **characterized in that** the main line (2) comprises, on the entry side of the system part (46) connected to the main line (2), an actuatable valve arrangement (35, 36) via which the liquid medium (45) is recirculatable, in particular back into the liquid storage container (48), preferably **in that** the valve arrangement (35, 36) is actuatable via control signals.

11. Bioprocessing system according to any of the preceding claims, **characterized in that** the bioprocessing system (1) comprises a control arrangement (38), and **in that** the powder transfer system (8) and/or the valve arrangement (35, 36) relating to recirculation and/or the main flow pump (5) is/are actuatable by the control arrangement (38).

12. Bioprocessing system according to claim 11, **characterized in that** a sensor arrangement (39) which ascertains a characteristic of the liquid medium (45) enriched by the additive (47) is provided, and **in that** the control arrangement (38) actuates the powder transfer system and/or the valve arrangement (35, 36) relating to recirculation and/or the main flow pump (5) depending on the sensor values of the sensor arrangement (39), preferably **in that** the characteristic ascertained is the concentration of the additive (47) in the liquid medium (45) and/or the temporal course of the concentration of the additive (47) in the liquid medium (45) and/or the input pressure or pressure profile, in particular in the main line (2).

13. Method for operation of a bioprocessing system (1) according to any of the preceding claims,
**characterized**
**in that** the additive (47) is fed into the feed line (9) in powder form by means of the powder transfer system (8) and it is driven toward the feed point (10) by means of a pressurized transport gas (12), and in that at least some of the fed additive (47) is delivered into the liquid medium (45) within the main line (2) downstream of the feed point (10) before the liquid medium (45) enriched with the additive (47) passes through or reaches the system part (46) connected to the main line (2).

14. Method according to claim 13, **characterized in that** the main flow pump (5) is operated such that the switching pressure gradient in the flow direction of the return flow preventer (11) is always fallen short of.

15. Use of a sterile-packed transfer set (42) composed of prefabricated system components for construction of a bioprocessing system (1) according to any of claims 1 to 12,
**characterized**
**in that** the transfer set in the sterile packaging comprises the feed line including the return flow preventer (11).

16. Use according to claim 15, **characterized in that** the transfer set (42) in the sterile packaging (43) additionally comprises the main line (2), preferably including the main flow pump (5), which is connected to the feed line (9) in a prefabricated manner at the feed point (10), preferably **in that** the transfer set (42) in the sterile packaging (43) comprises at least part of the liquid storage container (48), in particular of the bioreactor (14), in particular a bioprocess bag (15) assigned to the bioreactor (14), which is connected to the main line (2) in a prefabricated manner at the feed point (10).

17. Use according to claim 15 or 16, **characterized in that** the feed line (9) and/or the main line (2) and/or the return flow preventer (11) and/or the main flow pump (5), in particular as single-use components, is/are formed at least in part from a plastics material, preferably from a silicone material and/or PE (polyethylene), PP (polypropylene), PTFE (polytetrafluoroethylene), PBT (polybutylene terephthalate), PSU (polysulfone), PESU (polyether sulfone), PC (polycarbonate).

## Revendications

1. Installation technique de bioprocédé avec une conduite principale (2) pour la conduite d'un courant principal (3) dans un milieu liquide (45), en particulier biologique, avec une pompe de courant principal (5) pour la production du courant principal (3) et avec une pièce d'installation (46) raccordée à la conduite principale (2),
**caractérisée en ce que**
l'installation technique de bioprocédé (1) présente un système de transfert de poudre (8) pour la distribution dosée, en particulier sans poussière, d'un additif (47) et une conduite d'alimentation (9) raccordée au système de transfert de poudre (8), laquelle débouche en un emplacement d'alimentation (10) dans la conduite principale (2) et présente un empêcheur de reflux (11) pour empêcher un reflux de milieu liquide (45), **en ce que** le système de transfert de poudre (8) injecte sous forme de poudre l'additif (47) jusque dans la conduite d'alimentation (9) et le pousse vers l'emplacement d'alimentation (10) au moyen d'un gaz de transport (12) sous pression, et **en ce qu'**au moins une partie de l'additif (47) injecté est apportée dans le milieu liquide (45) à l'intérieur de la conduite principale (2) et en aval de l'emplacement d'alimentation (10), avant que le milieu liquide (45) enrichi avec l'additif (47) ne traverse ou n'atteigne la pièce d'installation (46) raccordée à la conduite principale (2).

2. Installation technique de bioprocédé selon la revendication 1, **caractérisée**
**en ce que** le milieu liquide (45) est un bouillon de fermentation (4), et/ou,
**en ce que** la pièce d'installation (46) raccordée à la conduite principale (2) est un élément fonctionnel technique de bioprocédé, de préférence un agencement de filtration (6) pour la filtration du bouillon de fermentation (4) ou un récipient de réception pour la réception du milieu liquide (45), et/ou,
**en ce que** l'additif (47) est un adjuvant de filtration (7) et/ou un sel et/ou un élément nutritif.

3. Installation technique de bioprocédé selon la revendication 1 ou 2,
avec une conduite principale (2) pour la conduite d'un courant principal (3) dans un bouillon de fermentation (4), avec une pompe de courant principal (5) pour la production du courant principal (3) et avec un agencement de filtration (6) raccordé à la conduite principale (2) pour la filtration du bouillon de fermentation (4),
dans laquelle l'installation technique de bioprocédé (1) présente un système de transfert de poudre (8) pour la distribution dosée, en particulier sans poussière, d'un adjuvant de filtration (7) et une conduite d'alimentation (9) raccordée au système de transfert de poudre (8), laquelle débouche en un emplacement d'alimentation (10) dans la conduite principale (2) et présente un empêcheur de reflux (11) pour empêcher un reflux de bouillon de fermentation (4), dans laquelle le système de transfert de poudre (8) injecte sous forme de poudre l'adjuvant de filtration (7) jusque dans la conduite d'alimentation (9) et le pousse vers l'emplacement d'alimentation (10) au moyen d'un gaz de transport (12) sous pression et dans laquelle au moins une partie de l'adjuvant de filtration (7) injecté est mis en suspension dans le bouillon de fermentation (4) à l'intérieur de la conduite principale (2) et en aval de l'emplacement d'alimentation (10), avant que le bouillon de fermentation (4) enrichi avec l'adjuvant de filtration (7) ne traverse l'agencement de filtration (6).

4. Installation technique de bioprocédé selon l'une des revendications précédentes, **caractérisée en ce que** l'installation technique de bioprocédé (1) présente un récipient collecteur de liquide (48), en particulier un bioréacteur (14), en particulier un bioréacteur (14) avec un sachet de bioprocédé (15) assigné, et **en ce que** le milieu liquide (45) est injecté depuis le récipient collecteur de liquide (48) jusque dans l'emplacement d'alimentation (10) via la conduite principale (2).

5. Installation technique de bioprocédé selon l'une des revendications précédentes, **caractérisée en ce que** la conduite d'alimentation (9) et/ou la conduite principale (2) et/ou l'empêcheur de reflux (11) et/ou la pompe de courant principal (5) est conçu(e) en particulier en tant que composante à usage unique, au moins en partie dans un matériau synthétique, de préférence dans un matériau en silicone et/ou en PE (polyéthylène), PP (polypropylène), PTFE (polytétrafluoroéthylène), PBT (polybutylène téréphtalate), PSU (polysulfone), PESU (polyéthersulfone), PC (polycarbonate).

6. Installation technique de bioprocédé selon l'une des revendications précédentes, **caractérisée en ce que** le système de transfert de poudre (8) présente une source de gaz de transport (17) pour le gaz de transport (12) sous pression et **en ce que** le système de transfert de poudre (8) injecte l'additif (47) jusque dans la conduite d'alimentation (9) au moyen du gaz de transport (12) sous pression, de préférence, **en ce que** le système de transfert de poudre (8) injecte en discontinu l'additif (47) jusque dans la conduite d'alimentation (9), ou, **en ce que** le système de transfert de poudre (8) injecte en continu l'additif (47) jusque dans la conduite d'alimentation (9).

7. Installation technique de bioprocédé selon l'une des revendications précédentes, **caractérisée en ce que** l'empêcheur de reflux (11) bloque un courant de fluide dans la direction du système de transfert de poudre (8) et, dans le cas d'un dépassement par le haut d'une chute de pression de commutation dans la direction de laisser-passer de l'empêcheur de reflux (11), laisse passer un courant dans l'additif, en particulier un courant d'adjuvant de filtration, dans la direction de la conduite principale (2), et/ou, **en ce que** l'empêcheur de reflux (11) est monté à l'instar d'un clapet anti-retour et présente un corps de clapet (31) élastique ou précontraint par ressort au moyen d'un agencement de ressort.

8. Installation technique de bioprocédé selon l'une des revendications précédentes, **caractérisée en ce que** la conduite d'alimentation (9) est en permanence exempte de milieu liquide (45) entre le système de transfert de poudre (8) et l'empêcheur de reflux (11), de préférence, **en ce que** la conduite d'alimentation (9) au moins dans le cas d'un courant principal stationnaire est en permanence exempte de milieu liquide (45) entre l'empêcheur de reflux (11) et l'emplacement d'alimentation (10).

9. Installation technique de bioprocédé selon l'une des revendications précédentes, **caractérisée en ce que** l'additif (47) est mis en suspension ou dissous dans le milieu liquide (45), de préférence, **en ce que** la mise en suspension ou la dissolution de l'additif (47) dans le milieu liquide (45) provient de l'écoulement de liquide dans la conduite principale (2) et/ou dans la pompe de courant principal, de préférence, **en ce que** la conduite principale (2) présente, en particulier en aval de l'emplacement d'alimentation (10), au moins une section de mélange et **en ce que** la conduite principale (2) présente, dans la section de mélange, des formations de mélange (34) sous la forme de profils de guidage d'écoulement, de briseurs d'écoulement ou similaire.

10. Installation technique de bioprocédé selon l'une des revendications précédentes, **caractérisée en ce que** la conduite principale (2) présente, du côté de l'entrée de la pièce d'installation (46) raccordée à la conduite principale (2), un agencement de clapets (35, 36) pouvant être piloté, via lequel le milieu liquide (45) peut être remis en circulation, en particulier retourné vers le récipient collecteur de liquide (48), de préférence, **en ce que** l'agencement de clapets (35, 36) peut être piloté via des signaux de commande.

11. Installation technique de bioprocédé selon l'une des revendications précédentes, **caractérisée en ce que** l'installation technique de bioprocédé (1) présente un agencement de commande (38) et **en ce que** le système de transfert de poudre (8) et/ou l'agencement de clapets (35, 36) concernant la remise en circulation et/ou la pompe de courant principal (5) peut ou peuvent être pilotés par l'agencement de commande (38).

12. Installation technique de bioprocédé selon la revendication 11, **caractérisée en ce qu'**est prévu un agencement de capteur (39) qui établit une caractéristique du milieu liquide (45) enrichi avec l'additif (47) et **en ce que** l'agencement de commande (38) pilote le système de transfert de poudre et/ou l'agencement de clapets (35, 36) concernant la remise en circulation et/ou la pompe de courant principal (5) en fonction des valeurs de capteur de l'agencement de capteur (39), de préférence, **en ce que** la caractéristique établie est, en particulier dans la conduite principale (2), la concentration de l'additif (47) dans le milieu liquide (45) et/ou l'évolution dans le temps de la concentration de l'additif (47) dans le milieu liquide (45) et/ou la pression d'entrée ou l'évolution de la pression.

13. Procédé d'exploitation d'une installation technique de bioprocédé (1) selon l'une des revendications précédentes,
**caractérisé**
**en ce que** l'additif (47) est injecté sous forme de poudre jusque dans la conduite d'alimentation (9) au moyen du système de transfert de poudre (8) et poussé vers l'emplacement d'alimentation (10) au moyen d'un gaz de transport (12) sous pression, et en ce qu'au moins une partie de l'additif (47) injecté est apportée dans le milieu liquide (45) à l'intérieur de la conduite principale (2) et en aval de l'emplacement d'alimentation (10), avant que le milieu liquide (45) enrichi avec l'additif (47) ne traverse ou n'atteigne la pièce d'installation (46) raccordée à la conduite principale (2).

14. Procédé selon la revendication 13, **caractérisé en ce que** la pompe de courant principal (5) est exploitée de sorte que la chute de pression de commutation dans la direction de laisser-passer de l'empêcheur de reflux (11) soit toujours inférieure.

15. Utilisation d'un kit de transfert (42) sous emballage stérile composé de composantes d'installation préassemblées pour le montage d'une installation technique de bioprocédé (1) selon l'une des revendications 1 à 12,
**caractérisé en ce que**
le kit de transfert dans l'emballage stérile présente la conduite d'alimentation y compris l'empêcheur de reflux (11).

16. Utilisation selon la revendication 15, **caractérisée en ce que** le kit de transfert (42) dans l'emballage stérile (43) présente en outre la conduite principale (2), de préférence y compris la pompe de courant principal (5), qui est raccordée déjà assemblée à l'emplacement d'alimentation (10) contre la conduite d'alimentation (9), de préférence, **en ce que** le kit de transfert (42) dans l'emballage stérile (43) présente au moins une partie du récipient collecteur de liquide (48), en particulier du bioréacteur (14), en particulier un sachet de bioprocédé (15) assigné au bioréacteur (14), lequel sachet de bioprocédé est raccordé en étant préassemblé à l'emplacement d'alimentation (10) contre la conduite principale (2).

17. Utilisation selon la revendication 15 ou 16, **caractérisée en ce que** la conduite d'alimentation (9) et/ou la conduite principale (2) et/ou l'empêcheur de reflux (11) et/ou la pompe de courant principal (5) est ou sont conçu(e)s en particulier en tant que composante à usage unique, au moins en partie dans un matériau synthétique, de préférence dans un matériau en silicone et/ou en PE (polyéthylène), PP (polypropylène), PTFE (polytétrafluoroéthylène), PBT (polybutylène téréphtalate), PSU (polysulfone), PESU (polyéthersulfone), PC (polycarbonate).
